(19) Europäisches Patentamt
European Patent Office
Office européen des brevets

(11) Publication number: **0 283 713 B1**

# (12) EUROPEAN PATENT SPECIFICATION

(45) Date of publication of patent specification: **11.08.93**

(51) Int. Cl.⁵: **C07F 9/10**, C07H 15/256, C07J 51/00, A61K 31/70, A61K 31/66, A61K 7/48

(21) Application number: **88102321.2**

(22) Date of filing: **18.02.88**

## (54) Complexes of saponins with phospholipids and pharmaceutical and cosmetic compositions containing them.

(30) Priority: **26.02.87 IT 1949687**

(43) Date of publication of application:
**28.09.88 Bulletin 88/39**

(45) Publication of the grant of the patent:
**11.08.93 Bulletin 93/32**

(84) Designated Contracting States:
**AT BE CH DE ES FR GB GR IT LI LU NL SE**

(56) References cited:
**DE-A- 1 217 547**

**CHEM.-BIOL. INTERACTIONS, vol. 52, 1984, pages 185-202, Elsevier Scientific Publishers Ireland Ltd, IR; B. SUL YU et al.: "Interaction of sea cucumber saponins with multilamellar liposomes"**

**CHEM.-BIOL. INTERACTIONS, vol. 56, 1983, pages 303-319, Elsevier Scientific Publishers Ireland Ltd, IR; B. SUL YU et al.: "Effects of purified ginseng saponins on multilamellar liposomes"**

(73) Proprietor: **INDENA S.p.A.**
**Via Ripamonti, 99**
**I-20141 Milano(IT)**

(72) Inventor: **Bombardelli, Ezio**
**Via Ripamonti, 99**
**I-20141 Milano(IT)**
Inventor: **Patri, Gian Franco**
**Via Ripamonti, 99**
**I-20141 Milano(IT)**
Inventor: **Pozzi, Roberto**
**Via Ripamonti, 99**
**I-20141 Milano(IT)**

(74) Representative: **Minoja, Fabrizio**
**Studio Consulenza Brevettuale Via Rossini, 8**
**I-20122 Milano (IT)**

EP 0 283 713 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**BIOCHIMICA ET BIOPHYSICA ACTA, vol. 820, 1985, pages 199-206, Elsevier Science Publishers B.V. (Biomedical Division), Amsterdam, NL; K. FUKUDA et al.: "Saponins can cause the agglutination of phospholipid vesicles"**

**E Bombardelli et al.: "Cosmetic utilization of complexes of Panax ginseng soponins with Phospholipids in Phytosome form". Fitoterapia vol.LX, suppl.al N.1-1989 pages 55-70**

## Description

The present invention relates to complexes of triterpene saponins, optionally complexed with cholesterol or phytosterols and with phospholipids, to a process for the preparation thereof and to pharmaceutical and/or cosmetic compositions containing them.

Saponins are triterpene compounds bonded with one or more sugar units, preferably pentacyclic triterpene deriving from oleanane, ursane, dammarane or triterpene possessing a five or six membered spirostanic ring in closed or open form.

Said saponins are extracted from known plants of pharmacologic interest, such as Aesculus ippocastanum, Centella asiatica, Ruscus aculeatus, Hedera helix, Terminalia sp., Calendula officinalis, Soya, Ginseng, Glycyrriza sp., Quillaia saponaria, Gypsophylla.

The present invention relates to complexes with the single saponins as well as complexes with the extracts from the plants containing said saponins. The invention relates also to complexes with some aglycons derived from the above mentioned saponins.

Examples of known and conveniently usable saponins according to the present invention comprise escin, sericoside, ginsenosides, ariunetine, ariunglycoside, asiaticoside, and their aglycons, asiatic acid, madecassic acid, sericic acid, oleanolic acid, hederin, glycirrethic acid, etc.

Said compounds are already known to possess some pharmacological properties which made them useful in human therapy. Particularly, the most investigated activities are antiedematous, vasotonic, vasoprotecting, immunomodulating and cutis tonifying activities.

Moreover, some of these compounds are active on cardiovascular system, central nervous system and can interfere in various enzyme and hormone systems.

Therapeutic use of saponins has been however restricted by several drawbacks, particularly:

1) poor absorption by oral route, partly due to fast bacterial degradation in the gastro-enteric tract, or to a complexation with cholesterol or biliary acids;

2) poor tolerability by cutaneous/topical administration, involving irritative phenomena of some seriousness;

3) remarkable toxicity by parenteral route, due to cytotoxicity and to a high haemolytic index, connected to an high affinity with cholesterol, with which they form complexes able to impair cell wall, which is generally rich in said compound.

Now it has surprisingly been found that complexes of saponins with phospholipids of natural or synthetic origin allow to overcome the above drawbacks, particularly allowing an effective absorption by oral and topical route and a high stability, due to the lipophilic characteristics attained.

Fukuda, K. et al. (Brochimica and Biophysica Acta 820 (1985) 199-206) investigate the interaction between saponin and phospholipidvesicles, the former causing the agglutination of the latter in aqueous buffers. From the investigation, only ginsenoside Rc resulted capable of agglutinating phospholipidvesicles. No data on chemical structure nor on therapeutic applicability are given.

Pharmacological activity and tolerability also turned out to be surprinsingly enhanced.

Instead of free single or admixed saponins, it is sometimes convenient to use complexes of saponins with cholesterol or phytosterols, which may be on their turn complexed with the phospholipids according to the present invention. This alternative proved to be particularly convenient for those saponins having a high hemolytic index or being markedly irritative for cutis and mucosae, said characteristics being effectively reduced or removed by the double complexation.

Complexes of saponins with cholesterol or phytosterols are generally known: more particularly, italian patent No. 22800 A/7 of April 28, 1978, discloses complexes of escin with cholesterol which are useful, inter alia, for isolating the saponins from the extracts containing them. Saponins from Hedera helix show some particular behavior to complexation: in fact, hederacosides form no complexes with cholesterol and require 1.5 to 2 moles of phospholipids per mole of saponin, whilst hederins form complexes with cholesterol and require substantially equimolecular ratios for the complexation with phospholipids.

The phospholipids that can be used according to this invention may be either vegetal or synthetic in nature, with acyl residues being the same or different, as shown by the formula:

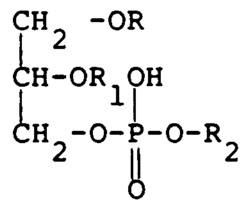

wherein R and $R_1$, which are the same or different, are mostly acyl residues of the palmitic, stearic, oleic, linoleic, linolenic acids, while $R_2$ is the residue of choline, phosphatidyl ethanolamine or serine. Particularly preferred phospholipids for use in cosmetics are the vegetale or naturally occurring phospholipids, such as those obtained from soy or from bovine or swine cutis or brain, similar to the ones that are found in human dermis; for other uses, a phospholipid which is chemically homogeneous and defined in its structure units (acyl and phosphorylamine groups) is preferred.

The complexes according to the invention are prepared by reacting the saponin, pure or in admixture with other components, such as in case of vegetal extracts, or the saponin/cholesterol or phytosterol complex, in an aprotic solvent with the phospholipid dissolved in the same solvent. The phospholipid/saponin or phospholipid/complex of saponin with cholesterol or phytosterol molar ratios are in the range from 0.5 to 2, more preferably about 1.

After solubilization has been completed, the complex compounds are isolated by removing the solvent under vacuum, by freeze drying or by precipitation with nonsolvents.

The thus obtained complexes are lipophilic in character and soluble in apolar and aprotic solvents, in which the individual components of the complex are normally insoluble.

The formation of a molecular complex is confirmed by a NMR spectroscopy study of the proton, carbon-13 and phosphorus, by comparing the spectra of the individual constituents with those of the reaction product. In the $^{1}H$-NMR spectrum of the complexes, the signals from the protons of the lipid chain are well evident, as well as a broadening of the band of N-$(Me)_3$ group of choline, showing that this moiety is involved in the complex compound.

In the $^{13}C$-NMR spectrum, the value of the relaxation times of the nuclei that are most involved in the complex's formation is reduced in a similar manner to the proton spectrum, until disappearance of all the signals characteristic of the terpene moiety of the saponin takes place.

In the $^{31}P$-NMR spectrum, a substantial broadening of the phosphorus band is observed with an evident peak shift. From these data, it can be deduced that, in the formation of the complex compound, the saponin are bound to the phospholipid by engagement with the polar head of this latter; the lipophilic character is imparted upon the complex compound by the lipid chains, which can freely rotate in the medium, as it can be deduced from their H-NMR spectrum, which shows no changes.

Some of the complexes compounds prepared according to the invention were pharmacologically tested: the complexed forms proved always to be more active than the corresponding free forms. Particularly, the complex of sericoside with distearoyl phosphatidylcholine proved to be more active than the sericoside in the carrageenin oedema test, after oral administration in the rat.

A higher activity was noted also after topical administration of an aqueous microdispersion of the complex of sericoside with soy phosphatidylcholine, in the same test.

Analogous results were obtained using complex compounds of saponins from Terminalia and Centella asiatica, topically administered on experimental wounds, in the vasal protection and capillary permeability tests as well as in the protecion test against UV radiations. The results which were obtained by means of the extracts were also confirmed using the single main components.

Experiments were also carried out using complex compounds of saponins from Hedera helix, of escine from Aesculus hippocastanum, and of ginsenosides (saponins from Panax ginseng). Particularly, the complexes of cholesterol/escin or B-sytosterol/escin with phospholipids, due to the increased lipophilic character, proved to be especially suited for the preparation of dermatological and cosmetic pharmaceutical formulations.

Finally, complexes of pure ginsenosides or mixtures thereof, besides showing an increased systemic biological activity connected to an improved absorption of the active ingredients, exhibit a moisturizing effect on the cutis, and make it more elastic when topically administered in cosmetics.

Said action is mainly due to a fibroblastic stimulation at the derma level, with a consequent increase in proteoglycan and collagene synthesis, which give tonicity to the cutis.

In all of the above cited cases, the complexes of the invention always turned out to be more active than non-complexed-saponins. Another interesting behaviour of these complexes when administered topically is the modification of the bioavailability, favouring the long lasting action. The table I reports the effect of Glycyrrhetinic acid complex with soy phospholipid standardised mixture (FITOSOMA[R]). From the data here reported it is evident that the action of Glycyrrhetinic acid complex is significantly prolonged in comparison with the equal quantity of the triterpene in free form.

**EVALUATION OF GLYCYRRHETINIC ACID/FITOSOMA**

| Substances | Dose /uM/ear | 6 h | 9 h | 12 h | 18 h | 24 h |
|---|---|---|---|---|---|---|
| Glycyr-rhetinic acid | 0.16 /uM | 83.9 | 57.6 | 50 | 33.5 NS | 23.5 NS |
| Glycyr-rhetinic acid/Fito-soma | 0.16 /uM | 77.4 | 71.2 | 74 | 69.2 | 82.4 S |
| Lipoid S-30 | 0.32 /uM | 53.2 | 57.6 | 51.9 | 53.8 | 22.4 MS |

Determinations were carried out at different times in mice.

From an applicative point of view of pharmaceutical and cosmetic technology, the various complex compounds obtained as above, can be employed as microdispersions in water by preparing them by homogenization using high-speed stirrers or ultrasonic procedures, or they may be incorporated as such into appropriate pharmaceutical or cosmetic preparations.

For topical administration, it is convenient to use the above mentioned microdispersion, which may be optionally added with thickening agents, said microdispersions can contain very wide percentages of active ingredients, from 0.1 to 30%, and may be also incorporated in forms of gels or emulsions for dermatologic or cosmetic purposes, or used as themselves as above mentioned. The complexes, due to their high lipophilia, may be dissolved in oils, in which they are stable, or incorporated in water/oil emulsions, or may be used in the preparation of capsules, tablets or suppositories.

In the preparation of the pharmaceutical compositions, care must be taken in using solvents, some of which, for examples alcohols and those having a high dielectric constant, such as dimethylsulfoxide, cleave the complexes, as evidenced by NMR spectroscopy. In fact, complexes dissolved in said solvents show spectra which substantially correspond to the summatory of spectra separately registered for the single constituents.

Therefore, in the formulations the compatibility of the compound with the dispersing medium must be taken into account, in order to safeguard complex stability and consequently activity.

Advantageously, in view of the higher activity of the complexed forms according to the invention, the active ingredient dosage may, under certain circumstances, be reduced, the specific activity remaining unchanged.

Suitable forms for pharmaceutical and/or cosmetic uses by topical application, are creams, gels or aqueous microdispersions containing 0.1 to 30% by weight of one or more complexes of the invention. Said

forms will be administered one or several times daily, depending on the intended use. Suitable forms for pharmaceutical uses, by oral administration, are tablets, capsules, syrups, granules, solutions, which contain unit doses of the complexed active principle in the range from 1 to 500 mg. Said pharmaceutical forms will be administered once or several times a day, depending on the severity of the pathology to be treated and the patient conditions.

The compositions according to the invention can in particular be used for treating conditions of inflammation, altered capillary fragility and permeability and, in general in all the fields in which an activity of the saponins is recognized at present.

In the following examples, that are given for illustrative purposes and not to be constructed as limiting the invention, use has been made of a soy phosphatidylcholine containing, on an average, as fatty acids: 63% linoleic acid, 16% palmitic acid, 3.5% steraric acid and 11% oleic acid based on the total fatty acids.

**EXAMPLE 1**

Complex of RG1 Ginsenoside with distearoylphosphatidylcholine

8.01 g of RG1 Ginsenoside were suspended in 15 ml of anhydrous methylene chloride and added with 7.9 g of synthetic distearoylphosphatidylcholine (titre: 99.9%); the suspension was then heated to mild reflux for 20 minutes or anyway until complete dissolution; the solution was concentrated to small volume and the concentrate was diluted with 60 ml of n-hexane. A white precipitate was obtained which, after filtration and drying at 40° C, weighed 15 g; m.p. 127-130°C; $[\alpha]_D$ + 16.82 (C = 0.3 $CHCl_3$/ MeOH 1:1).

NMR and IR confirm the formation of the complex.

**EXAMPLE 2**

Complex of RB1 Ginsenoside with distearoylphosphatidylcholine

11 g of RB1 Ginsenoside were treated as described in Example 1 with 7.9 g of distearoylphosphatidylcholine. After precipitation and drying, 18.5 g were obtained, having m.p. 129-132°C and $[\alpha]_D$ + 6.50 (C = 0.3 $CHCl_3$/MeOH 1:1).

**EXAMPLE 3**

Complex of Rd Ginsenoside with distearoylphosphatidylcholine

9.4 g of Rd Ginsenoside were treated, as in Example 1, with 7.9 g of synthetic distearoylphosphatidylcholine (titre 99.9%). After precipitation and drying, 17 g of complex were obtained, having m.p. 178°C.

**EXAMPLE 4**

8.01 g of RG1 Ginsenoside were treated, as in Example 1, with 7.8 g of soy phosphatidylcholine (titre 95% in phosphatidylcholine). After precipitation and thorough washing of the residue with n-hexane, to remove the excess phospholipide, and drying at 40°C under vacuum, 15 g of the complex were obtained, having m.p. 107 - 109°C and spectroscopic characteristics corresponding to a complex.

**EXAMPLE 5**

Preparation of the complex of total saponins extracted from Panax Ginseng M with soy phosphatidylcholine.

5 g of total saponins extracted from the main roots of Panax Ginseng M, having a titre in RG1 Ginsenoside and RB1 Ginsenoside of 18% and 40%, respectively, the remaining consisting of other ginsenosides (Re, Rc, b2, Rf, Ro) were suspended in anhydrous acetone and added with 7 g of soy phoshatidylcholine (titre 95% in phosphatidylcholine) dissolved at mild reflux in 30 ml of methylene chloride. The suspension was heated to mild reflux until complete dissolution.

The so obtained solution, after optional filtration of any insoluble impurities, was concentrated to small volume; the residue was diluted with 50 ml more of acetone, which were re-distilled. The acetone residue after distillation, was diluted with 100 ml of n-hexane. The formed precipitate was filtered and thoroughly

washed with a 75:5 n-hexane/acetone mixture, to remove the excess lipid. After drying 10 g of a beige powder were obtained, having m.p. 102 - 103 °C and spectroscopic characteristics in agreement with the formation of a complex.

**EXAMPLE 6**

Preparation of the complex of sericoside with distearoylphosphatidylcholine

3.3 g of sericoside were suspended in 10 ml of methylene chloride and added with 4 g of distearoylphosphatidylcholine.
The suspension was heated for some minutes, until complete dissolution, then the solution was concentrated to small volume. The residue was taken up in 30 ml of n-hexane; a white precipitate formed which, upon filtration and drying, had m.p. 144 - 146 °C and $[\alpha]_D$ + 9.9 ° (C = 0.5 $CHCl_3$/MeOH 1:1).

**EXAMPLE 7**

Preparation of the complex of ariunetin with soy phosphatidylcholine

3.2 g of ariunetin were treated, as in Example 6, with 4.5 g of soy phosphatidylcholine (titre 95%). After filtration and drying, 7 g of the complex were obtained, melting at 152 °C.

**EXAMPLE 8**

Preparation of the complex of ariunglycosyde with soy phosphatidylcholine

3.3 g of ariunglycosyde were treated as in Example 6, with 4.5 g of soy phosphatidylcholine (titre 95%). Upon filtration, washing and drying, 7.1 g of the complex were obtained, having m.p. 115-118 °C.

**EXAMPLE 9**

Complex of a triterpene fraction from Terminalia sericea with soy phosphatidylcholine

6.5 g of triterpene fraction from Terminalia sericea Burch, comprising 65% sericoside, 28% ariunetine and 6% ariunglycoside, were suspended in 50 ml of anhydrous acetone and added with 30 ml of a dichloromethylene solution containing 8 g of soy phosphatidylcholine (titre: 95% in phosphatidylcholine). The suspension was heated to mild reflux until complete dissolution. Methylene chloride and a part of acetone were removed by distillation. The distillation residue was poured into 100 ml of n-hexane and after filtration and drying of the precipitate, 13.5 g of a white-yellowish powder were obtained, which was completely soluble in apolar solvents and had spectroscopic characteristics confirming the formation of the complex.

**EXAMPLE 10**

Preparation of the complex of a terpene fraction from Terminalia sericea with soy phosphatidylcholine

6.5 g of a terpene fraction from Terminalia sericea Burch containing 90% sericoside, 6% ariunetine and 5% ariunglycoside were dissolved together with 8 g of soy phosphatidylcholine (titre 95%) in 25 ml of dioxane.

By freeze-drying the solution a white amorphous powder which was soluble in oils and aprotic organic solvents, and had the spectroscopic characteristics corresponding to those of a complex, was obtained.

**EXAMPLE 11**

Preparation of the complex of a terpene fraction prepared from Centella asiatica with soy phosphatidylcholine

8 g of a mixture consisting of 40% asiaticoside, 30% of asiatic acid and 30% of madecassic acid, were dissolved in 50 ml of dioxane, together with 9 g of soy phosphatidylcholine (titre 95%). The resulting

solution was freezedried. 16.5 g of complex of saponins with triterpene acids were obtained, whose solubility and spectroscopic data were in agreement with the complex. M.p. of the complex 182-186 °C.

**EXAMPLE 12**

Preparation of the complex of asiaticoside with distearoylphosphatidylcholine

9.5 g of asiaticoside were suspended in 50 ml of methylene chloride and added with 9 g of distearoylphosphatidylcholine (titre: 99.9%). The suspension was heated to mild reflux until complete dissolution. The chloromethylene solution was concentrated to small volume and poured into 200 ml of n-hexane. After filtration and drying, 17.5 g of a white powder having spectroscopic characteristics in agreement and m.p. 187 °C and $[\alpha]_D$ + 2 (C = 0.25 $CHCl_3$/MeOH 1:1).

**EXAMPLE 13**

Preparation of the complex of the saponins from Edera helix with soy phosphatidylcholine

5 g of Hedera saponins having 90% of ederin were treated with 8 g of soy phosphatidylcholine(titre 95%) in 30 ml of dioxane. The solution was freeze-dried to obtain 12.5 g of the complex.

**EXAMPLE 14**

5 g of the complex of saponins from Edera helix with cholesterol (titre 45% in ederine) were dissolved in 150 ml of anhydrous dioxane, together with 10 g of soy phosphatidylcholine (titre 95%). The solution was freeze-dried, to obtain 15 g of a product which was easily dispersible in water by treatment wiht Ultraturax (stirrer at 30.000 rpm).

**EXAMPLE 15**

Preparation of the complex of total saponins from Ruscus aculeatus (titre 70%) with soy phosphatidylcholine

10 g of total saponins with a 70% titre were dissolved with 15 g of soy phosphatidylcholine, under the conditions of Example 12, 14 g of white-beige product were obtained, which had chemico-physical and spectroscopic characteristics in agreement with the title complex.

**EXAMPLE 16**

Preparation of the complex of saponins from Calendula officinalis with soy phosphatidylcholine

5 g of saponins from Calendula officinalis with titre expressed in oleanic acid and ederagenin of 8%, were treated, according to Example 15, with 8 g of phosphatidylcholine. A lipophilic compound was obtained, which was soluble in oils, in which it could be directly used.

**EXAMPLE 17**

Preparation of the complex of 18 Beta-glycirrethic acid with soy phosphatidylcholine

4.7 g of 18 Beta-glycirrethic acid were suspended together with 8.8 g of soy phosphatidylcholine (lipoid S-100) in 100 ml of methylene chloride, heating the mixture to mild reflux. When dissolution was complete, solvent was removed under vacuum. 13.5 g of a vitrous solid were obtained, having $[\alpha]^{20}$ + 32 ° (C = 1% in $CHCl_3$) and m.g. 184-188 °C.

**EXAMPLE 18**

Preparation of a microdispersion, starting from the complex of the saponins from Centella asiatica of Example 11

400 ml of distilled water were placed into a 600 ml vessel, and 6 g of complex of selected triterpenes from Centella asiatica with phosphatidylcholine were added under strong mechanic stirring, by means of Ultra® or equivalents. The suspension was kept under stirring after the addition was complete, for 30 minutes. A stabile milky microdispersion was obtained, to which 0.6 g of a thickening agent and 0.6 ml of Katon® solution as a preserver were added.
Such suspension may be used as such for topical administration.

**EXAMPLE 19**

Anhydrous gel containing the complex beta-sitosterol/escin/phosphatidylcholine

100 g of gel contain:

| | |
|---|---|
| Beta-sitosterol-escin-phosphatidylcholine compl. | 3 g |
| Tween 20 | 29 g |
| Alcohol 95% | 15 g |
| Propylene glycole | 49 g |
| Carbopol 934 | 3 g |
| Parfum | 0.9 g |
| Preservants | 0.1 g |

**EXAMPLE 20**

Gel containing the complex sericosyde/soy phosphatidylcholine

100 g of gel contain:

| | |
|---|---|
| Sericosyde/soy phosphatidylcholine (Lipoid S-100) | 1.5 g |
| Kathon | 0.1 g |
| Imidazolidine-urea | 0.3 g |
| Ethoxylated $C_8$-$C_{12}$ triglycerides | 25 g |
| Poyoxyethylen-20-deiletere | 6 g |
| Carboxyvinyl-polymer | 1.5 g |
| Triethanolamine | 2 g |
| Parfum | 0.2 g |
| Distilled water | 63.4 g |

## Claims

**1.** Phospholipid complexes of saponins, which may be on their turn optionally complexes with cholesterol or phytosterols.

**2.** Complexes according to claim 1, wherein the phospholipide /saponin ratio is comprised from 0.5 to 2.

**3.** Complexes according to claim 2, wherein the phospholipide/saponin ratio is about 1.

**4.** Complexes according to any one of the preceeding claims, wherein the saponins derive from Aesculus ippocastanum, Centella asiatica, Ruscus aculeatus, Hedera helix, Terminalia sp., Calendula officinalis, Soya, Panax Ginseng, Glycyrriza sp., Quillaia saponaria, Gypsophylla and their aglycons.

5. Complexes according to any one of the preceeding claims, wherein the saponin is selected from the group consisting of escin, sericoside, ginsenosides, ariunetine, ariunglycoside, asiaticoside, asiatic acid, madecassic acid, hederin, glycirrethic acid.

6. Complexes according to any one of the previous claims, wherein the phospholipids used are selected in the group consisting of soy lecithins or egg, phospholipids from bovine or swine brain or dermis, phosphatidyl choline, phosphatidyl serine, phosphatidyl ethanolamine in which the acyl groups may be the same or different and are mostly derived from palmitic, stearic, oleic, linoleic, linolenic acids.

7. Substantially equimolar complex of soy phosphatidylcholine with escin/cholesterol or $\beta$-sitosterol.

8. Complexes according to claims 1-7, for use as medicaments.

9. Complexes according to claim 1-7, for use in dermatologic and cosmetic field.

10. Pharmaceutical or cosmetic compositions containing as the active principle a complex of claims 1-7, in admixture with a suitable carrier.

11. Composition according to claim 10 for topical applications in form of acqueous microdispersions and optionally added with thickening agents for the preparations of gels or creams.

**Patentansprüche**

1. Phospolipidkomplexe mit Saponinen, die ihrerseits gegebenenfalls mit Cholesterin oder Phytosterinen Komplexe bilden.

2. Komplexe nach Anspruch 1, dadurch **gekennzeichnet,** daß das Phospholipid/Saponinverhältnis 0,5 bis 2 beträgt.

3. Komplexe nach Anspruch 2, dadurch **gekennzeichnet,** daß das Phospolipid/Saponinverhältnis etwa 1 beträgt.

4. Komplexe nach einem der vorausgehenden Ansprüche, dadurch **gekennzeichnet,** daß sich die Saponine von Aesculus ippocastanum, Centella asiatica, Ruscus aculeatus, Hedera helix, Terminalia sp., Calendula officinalis, Soja, Panax Ginseng, Glycyrriza sp., Quillaia saponaria, Gypsophylla und ihren Aglycons ableiten.

5. Komplexe nach einem der vorausgehenden Ansprüche, dadurch **gekennzeichnet,** daß das Saponin aus der Gruppe bestehend aus Aescin, Sericosid, Ginsenosiden, Ariunetin, Ariunglycosid, Asiaticosid, Asiaticasäure, Madecassasäure, Hederin, Glycyrrethinsäure ausgewählt ist.

6. Komplexe nach einem der vorausgehenden Ansprüche, dadurch **gekennzeichnet,** daß die verwendeten Phospholipide aus der Gruppe bestehend aus Sojalecithinen oder Eiern, Phospholipiden aus Rinder- oder Schweinehirn oder -haut, Phosphatidylcholin, Phosphatidylserin, Phosphatidylethanolamin, worin die Acylgruppen gleich oder verschieden sein können und sich meistens von Palmitin-, Stearin-, Öl-, Linol-, Linolensäuren ableiten, ausgewählt sind.

7. Im wesentlichen equimolarer Komplex aus Sojaphosphatidylcholin mit Aescin/Cholesterin oder $\beta$-Sitosterin.

8. Komplexe nach den Ansprüchen 1 bis 7 zur Verwendung als Medikamente.

9. Komplexe nach Anspruch 1 bis 7 zur Verwendung auf dem dermatologischen und kosmetologischen Gebiet.

10. Pharmazeutische oder kosmetische Präparate, enthaltend als Wirkstoff einen Komplex nach den Ansprüchen 1 bis 7 im Gemisch mit einem geeigneten Träger.

**11.** Präparat nach Anspruch 10 für topische Anwendungen in Form wäßriger Microdispersionen und gegebenenfalls versetzt mit Verdickungsmitteln zur Herstellung von Gelen oder Cremes.

**Revendications**

**1.** Complexes phospholipidiques de saponines, qui peuvent elles-mêmes facultativement être des complexes avec le cholestérol ou des phytostérols.

**2.** Complexes selon la revendication 1, dans lesquels le rapport phospholipide/saponine est compris entre 0,5 et 2.

**3.** Complexes selon la revendication 2, dans lesquels le rapport phospholipide/saponine est d'environ 1.

**4.** Complexes selon l'une quelconque des revendications précédentes, dans lesquels les saponines sont des saponines provenant de *Aesculus hippocastanum, Centella asiatica, Ruscus aculeatus, Hedera helix, Terminalia* sp., *Calendula officinalis,* soja, *Panax Ginseng, Glycyrrhiza* sp., *Quillaya saponaria, Gypsophila* et leurs aglycones.

**5.** Complexes selon l'une quelconque des revendications précédentes, dans lesquels la saponine est choisie dans le groupe formé par l'aescine, le séricoside, les ginsénosides, l'arjunétine, l'arjunglycoside, l'asiaticoside, l'acide asiatique, l'acide madécassique, l'hédérine, l'acide glycyrrhétique.

**6.** Complexes selon l'une quelconque des revendications précédentes, dans lesquels les phospholipides utilisés sont choisis dans le groupe formé par les lécithines de soja ou d'oeuf, les phospholipides provenant du cerveau ou du derme de bovin ou de porc, la phosphatidylcholine, la phosphatidylsérine, la phosphatidyléthanolamine dont les groupes acyles peuvent être identiques ou différents et sont principalement dérivés des acides palmitique, stéarique, oléique, linoléique, linolénique.

**7.** Complexe sensiblement équimolaire de phosphatidylcholine de soja avec l'aescine/cholestérol ou $\beta$-sitostérol.

**8.** Complexes selon les revendications 1 à 7, pour leur utilisation comme médicaments.

**9.** Complexes selon les revendications 1 à 7, pour leur utilisation dans les domaines dermatologique et cosmétique.

**10.** Compositions pharmaceutiques ou cosmétiques contenant un complexe des revendications 1 à 7 comme principe actif, en mélange avec un support approprié.

**11.** Compositions selon la revendication 10, pour des applications topiques sous forme de microdispersions aqueuses et facultativement additionnées d'épaississants pour les préparations en gel ou en crème.